# EUROPEAN PATENT APPLICATION

(11) **EP 4 252 727 A1**
(43) Date of publication of application: **04.10.2023**
(21) Application number: 21897535.7
(22) Date of filing: 15.10.2021
(51) Int. Cl.: A61F 13/511, A61F 13/514, A61F 13/515, A61F 13/53, A61F 13/532

(54) **ABSORBENT ARTICLE**

(30) Priority: 30.11.2020 JP 2020197890
(71) Applicant: Unicharm Corporation, Ehime 799-0111 (JP)
(72) Inventor: GODA, Hiroki, Kanonji-shi, Kagawa 769-1602 (JP); TSUKUDA, Atsushi, Kanonji-shi, Kagawa 769-1602 (JP)
(74) Representative: Staeger & Sperling Partnerschaftsgesellschaft mbB
(86) International application number: PCT/JP2021/038209
(87) International publication number: WO 2022/113557

(57) **Abstract**

This absorbent article (1) has a vertical direction, lateral direction, and thickness direction perpendicular to each other and has an absorbent body (11) comprising an absorbent core (12), a first sheet part (13bp) that covers the absorbent core (12) from one side in the thickness direction, and a second sheet part (13ap) that covers the absorbent core (12) from the other side in the thickness direction. The absorbent article (1) is characterized in that the absorbent core (12) includes a through-hole (12H) penetrating in the thickness direction and has, inside the through-hole (12H) in a plan view, a first joining part and second joining part at which the first sheet part (13bp) and the second sheet part (13ap) are joined, and the second joining part is separated from the first joining part by a predetermined distance in the lateral direction.

## Description

### FIELD

The present invention relates to an absorbent article.

### BACKGROUND

Generally, it is widely known that an absorbent article has an absorbent body, and that the absorbent body includes an absorbent core and a core-wrapping sheet which covers the absorbent core. For example, Patent Literature 1 discloses an absorbent article as follow: a non-fiber-deposition portion that penetrates the absorbent core in the thickness direction is provided in order to enhance the diffusibility of excreted fluid and to facilitate to deform the absorbent core so as to follow the wearer's body; and further a skin-side core-wrapping sheet and a non-skin-side core-wrapping sheet are joined in the non-fiber-deposition portion to enhance the shape retention of the absorbent core in order to enhance the shape retention of the absorbent core.

### [CITATION LIST]

### [PATENT LITERATURE]

Patent Literature 1: Japanese Patent Application Publication No. 2016-083194

### SUMMARY

### [TECHNICAL PROBLEM]

However, in the absorbent article of Patent Literature 1, in the non-fiber-deposition portion, a portion where the skin-side core-wrapping sheet and the non-skin-side core-wrapping sheet are joined is joined with an adhesive or the like. This causes a risk of the decrease of the absorbency or a risk of the decrease of the diffusibility of excrement in the non-fiber-deposition portion.

The present invention was achieved in light of problems that described above and an aspect of the present invention is to provide an absorbent article in which absorbency is enhanced and the diffusion of excrement in a longitudinal direction is facilitated, while enhancing the followability to the wearer's body and the shape retention of the absorbent core.

### [SOLUTION TO PROBLEM]

A main aspect of the present invention for achieving the above-described aspect is an absorbent article having a longitudinal direction, a lateral direction, and a thickness direction that are orthogonal to each other, the absorbent article including: an absorbent body including an absorbent core, a first sheet portion that covers the absorbent core from a one side in the thickness direction, and a second sheet portion that covers the absorbent core from another side in the thickness direction, the absorbent core including a through hole that penetrates in the thickness direction, the absorbent article has a first joining portion and a second joining portion inside the through hole in a plan view, each of the first joining portion and the second joining portion being a portion in which the first sheet portion and the second sheet portion are joined, the second joining portion being spaced apart from the first joining portion in the lateral direction by a predetermined distance in the plan view.
Features of the present invention other than the above will become clear by reading the description of the present specification with reference to the accompanying drawings.

### [ADVANTAGEOUS EFFECT OF THE INVENTION]

According to the present invention, it is possible to provide an absorbent article in which the diffusion of excrement in a longitudinal direction is facilitated, while enhancing the followability to the wearer's body and the shape retention of the absorbent core.

### BRIEF DESCRIPTION OF DRAWINGS

FIG. 1 is a schematic perspective view of an underpants-shaped disposable diaper 1.
FIG. 2 is a schematic plan view of the diaper 1 in an unfolded and stretched state when viewed from a skin-side surface.
FIG. 3 is a schematic cross-sectional view taken along arrows A-A in FIG. 2.
FIG. 4 is an explanatory diagram illustrating a non-skin-side sheet 13b.
FIG. 5A is an enlarged view of a portion X in FIG. 3.
FIG. 5B is an enlarged view of a portion Y in FIG. 5A.
FIG. 6A is an explanatory diagram illustrating an application region of an adhesive g of the diaper 1.
FIG. 6B is an explanatory diagram illustrating an application region of the adhesive g of the diaper 1 in another embodiment.
FIG. 6C is an explanatory diagram illustrating an application region of the adhesive g of the diaper 1 in another embodiment.
FIG. 7 is a schematic cross-sectional view taken along an arrow B-B of an absorbent body 11 in FIG. 2.
FIG. 8A is an enlarged view of a portion according to another embodiment, corresponding to the portion X in FIG. 3.
FIG. 8B is an enlarged view of a portion according to still another embodiment, corresponding to the portion X in FIG. 3.
FIG. 8C is an enlarged view of a portion according to still another embodiment, corresponding to the portion X in FIG. 3.

### DESCRIPTION OF EMBODIMENTS

At least following matters will become clear with description of this specification and attached drawings.
An absorbent article having a longitudinal direction, a lateral direction, and a thickness direction that are orthogonal to each other, the absorbent article including: an absorbent body including an absorbent core, a first sheet portion that covers the absorbent core from a one side in the thickness direction, and a second sheet portion that covers the absorbent core from another side in the thickness direction, the absorbent core including a through hole that penetrates in the thickness direction, the absorbent article has a first joining portion and a second joining portion inside the through hole in a plan view, each of the first joining portion and the second joining portion being a portion in which the first sheet portion and the second sheet portion are joined, the second joining portion being spaced apart from the first joining portion in the lateral direction by a predetermined distance in the plan view.

According to the above-described absorbent article, by making the absorbent core more likely to deform due to providing the through hole, the followability to the wearer's body is enhanced. While enhancing the shape retention of the absorbent core, the absorbency is enhanced due to providing a portion in which the first sheet portion and the second sheet portion are not joined in the through hole. This facilitates the diffusion of excrement in the longitudinal direction.

In such an absorbent article, it is desirable that inside the through hole, the first sheet portion has a plurality of protruding portions protruding in the thickness direction, at least on a surface of the first sheet portion that faces the second sheet portion, and that the protruding portions and the second sheet portion are joined by each of the first joining portion and the second joining portion.

According to the above-described absorbent article, the first sheet portion having the plurality of protruding portions makes it easier to provide a space in a region where the first joining portion and the second joining portion corresponding to each of the protruding portions are spaced apart from each other in the lateral direction. The space provided between the first joining portion and the second joining portion in the lateral direction enhances absorbency, making excrement more likely to move in the longitudinal direction.

In such an absorbent article, it is desirable that the first sheet portion has the plurality of protruding portions on two side surfaces in the thickness direction.

According to the above-described absorbent article, providing the protruding portions on the two side surfaces of the first sheet portion makes excrement more likely to diffuse in the longitudinal direction not only on the surface that faces the second sheet but also on the surface opposite to the surface facing the second sheet portion.

In such an absorbent article, it is desirable that a fiber density of a central portion of the protruding portion in the lateral direction is higher than a fiber density of a side end portion of the protruding portion in the lateral direction.

According to the above-described absorbent article, the lateral central portion of the protruding portion is more likely to become a position corresponding to the first joining portion. In addition, the joining between the first joining portion and the second joining portion can be further strengthened compared with the case where the fiber density of the lateral central portion of the protruding portion is lower than the fiber density of the lateral side end portion of the protruding portion.

In such an absorbent article, it is desirable that inside the through hole, an average height of the protruding portions located on a surface of the second sheet portion that faces the first sheet portion is lower than an average height of the protruding portions located on a surface of the first sheet portion that faces the second sheet portion.

According to the above-described absorbent article, the joining between the first joining portion and the second joining portion can be further strengthened compared with the case where the second sheet portion has protruding portions to the same extent of the first sheet portion.

In such an absorbent article, it is desirable that inside the through hole in the plan view, the first sheet portion has a plurality of first sheet protruding portions protruding in the thickness direction, on a surface of the first sheet portion that faces the second sheet portion, that inside the through hole in the plan view, the second sheet portion has a plurality of second sheet protruding portions protruding in the thickness direction, at least on a surface of the second sheet portion that faces the first sheet portion, and that the first sheet protruding portions and the second sheet portion are joined by each of the first joining portion and the second joining portion, or the second sheet protruding portions and the first sheet portion are joined by each of the first joining portion and the second joining portion.

According to the above-described absorbent article, since the first sheet portion and the second sheet portion each have the protruding portions, it is possible to increase the thickness of the sheet portions. This makes excrement more likely to be absorbed in the through hole, making it possible to reduce the risk that the absorbed excrement returns to the skin side.

In such an absorbent article, it is desirable that inside the through hole, the absorbent article has the first joining portion, the second joining portion, and another joining portion in which, and at least one or more portions of the first sheet portion and the second sheet portion are joined, and that the other joining portion is spaced apart from the first joining portion and the second joining portion in the lateral direction.

According to the above-described absorbent article, while further strengthening the joining between the first sheet portion and the second sheet portion inside the through hole, excrement is more likely to diffuse in the longitudinal direction in a region where the first joining portion, the second joining portion, and the other joining portion are separated apart from each other.

In such an absorbent article, it is desirable that the through hole extends along the longitudinal direction, that inside the through hole, the first sheet portion has a plurality of protruding portions on a surface of the first sheet portion that faces at least the second sheet portion, the plurality of protruding portions coming into contact with the second sheet portion and protruding in the thickness direction, and that two protruding portions that are laterally adjacent to each other are spaced apart in the lateral direction and extend along the longitudinal direction.

According to the above-described absorbent article, inside the through hole having a shape extending along the longitudinal direction, the protruding portions of the first sheet portion that are in contact with the second sheet portion extend along the longitudinal direction, and therefore a region between the first joining portion and the second joining portion in the lateral direction is likely to become a region that extends along the longitudinal direction, making excrement more likely to diffuse in the longitudinal direction inside the through hole.

In such an absorbent article, it is desirable that inside the through hole, a longitudinal distance at which the two protruding portions are in contact with the second sheet portion is longer than the predetermined distance.

According to the above-described absorbent article, compared with the case where the longitudinal distance at which the two protruding portions are in contact with the second sheet portion is equal to or shorter than the distance between the first joining portion and the second joining portion in the lateral direction (predetermined distance), the region between the first joining portion and the second joining portion in the lateral direction is made more likely to maintain its shape extending along the longitudinal direction. This makes excrement more likely to diffuse in the longitudinal direction.

In such an absorbent article, it is desirable that the one side in the thickness direction is a non-skin side, that the other side in the thickness direction is a skin side, and that the first sheet portion is a non-skin-side sheet portion that is located on the non-skin side with respect to the absorbent core.

According to the above-described absorbent article, the non-skin-side sheet portion located on the non-skin side with respect to the absorbent core has the first joining portion and the second joining portion that are spaced apart from each other in the lateral direction by the predetermined distance. This makes the excrement absorbed from the skin side of the through hole more likely to remain in the region in which the first joining portion and the second joining portion are spaced apart from each other. Therefore, it is more likely to reduce the risk that the excrement once absorbed by the absorbent body returns to the wearer's skin side.

In such an absorbent article, it is desirable that the first joining portion and the second joining portion are provided on the skin side with respect to a center of the absorbent core in the thickness direction.

According to the above-described absorbent article, compared with the case where the first joining portion and the second joining portion are provided at the center in the thickness direction, the absorbent body is recessed more deeply from the non-skin-side surface toward the skin side and therefore the presence of the through hole is likely to be recognized when the wearer views the absorbent article from the non-skin side.

In such an absorbent article, it is desirable that an adhesive is provided in at least either one of the first sheet portion and the second sheet portion, and that the adhesive is provided in a manner of continuing in the lateral direction.

According to the above-described absorbent article, the first joining portion and the second joining portion are more likely to be formed. In addition, in the region where the first joining portion and the second joining portion are spaced apart from each other, the absorbency is enhanced, and excrement is more likely to diffuse in the longitudinal direction.

In such an absorbent article, it is desirable that in the lateral direction, a spacing distance between the first joining portion and the second joining portion is shorter than a length obtained by dividing by two a sum of a length of the first joining portion and a length of the second joining portion.

According to the above-described absorbent article, compared with the case where the spacing distance between the first joining portion and the second joining portion is longer than the length obtained by dividing by two the sum of the length of the second joining portion and the length of the second joining portion, it is possible to make larger the regions of the joining portions. This can further strengthen the joining between the first sheet portion and the second sheet portion.

In such an absorbent article, it is desirable that in the lateral direction, a spacing distance between the first joining portion and the second joining portion is longer than a length obtained by dividing by two a sum of a length of the first joining portion and a length of the second joining portion.

According to the above-described absorbent article, compared with the case where the spacing distance between the first joining portion and the second joining portion is shorter than the length obtained by dividing by two the sum of the length of the second joining portion and the length of the second joining portion, it is possible to narrow the regions of the joining portions. This can decrease the stiffness of the region of the absorbent body in which the through hole is provided, making the absorbent body more likely to deform from the through hole and to follow the wearer's body.

In such an absorbent article, it is desirable that at least longitudinal one-side ends of the first sheet portion and the second sheet portion are positioned outside a longitudinal one-side end of the absorbent core, and that, on a one side in the longitudinal direction, an end portion of the first sheet portion and an end portion of the second sheet portion are joined continuing in the lateral direction.

According to the above-described absorbent article, the region where the first joining portion and the second joining portion are spaced apart from each other can reduce the risk that excrement that has diffused in the longitudinal direction leaks out from the longitudinal end portion of the absorbent body.

In such an absorbent article, it is desirable that the absorbent body includes a pair of the through holes at a central portion when the absorbent body is divided into three portions in the longitudinal direction, and that the pair of through holes are provided on a one side and another side of the absorbent body in the lateral direction.

According to the above-described absorbent article, the absorbent article is easily arranged in the wearer's crotch, making the absorbent body more likely to deform according to the movement of the wearer.

### Embodiments

The following describes an absorbent article using an absorbent body according to the present embodiment by way of example of a so-called underpants-shaped disposable diaper (hereinafter, also referred to as a "diaper 1"). It should be noted that the absorbent article is not limited to an underpants-shaped disposable diaper, and examples thereof include a tape-type disposable diaper, a sanitary napkin, an absorbent pad, a disposable diaper for pets, and an absorbent pad for pets. The disposable diaper, absorbent pad, or the like can be used for infants or adults. It should be noted that excrement refers to excrement of living body, including not only humans but also animals. Examples of excrement include sweat, urine, feces, menstrual blood, vaginal discharge, breast milk, blood, and exudate liquid.

### Basic configuration of underpants-shaped disposable diaper 1

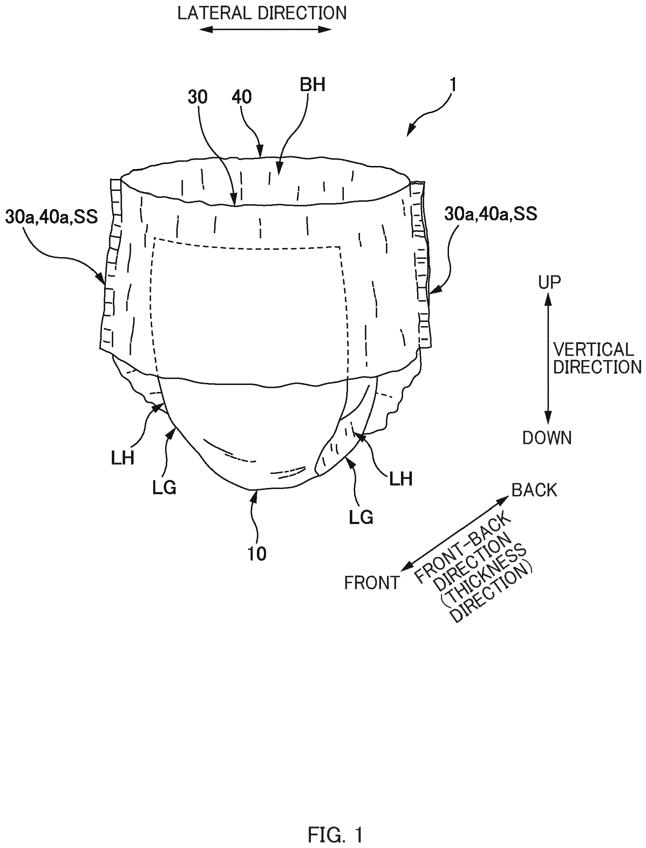

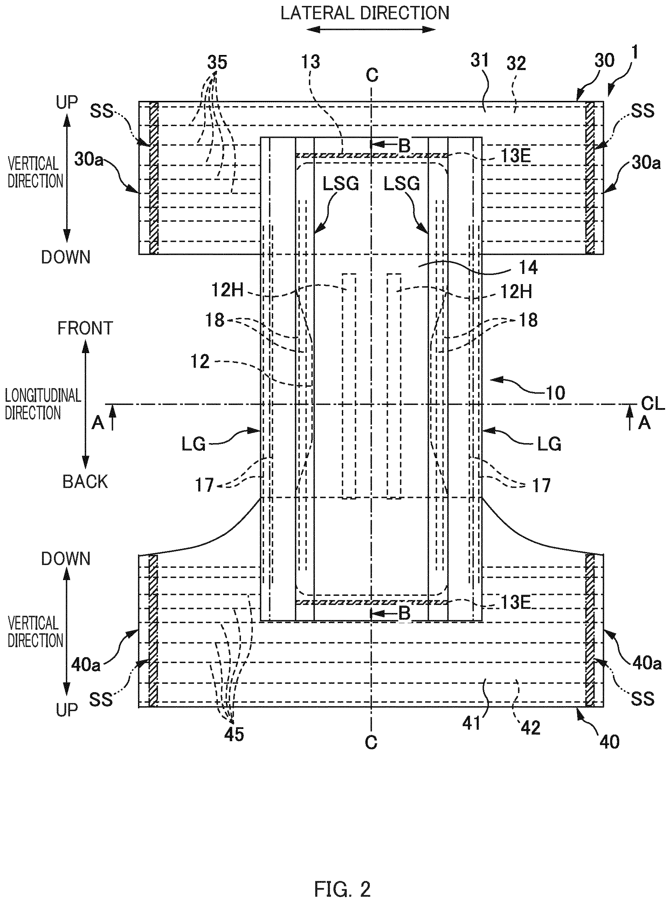

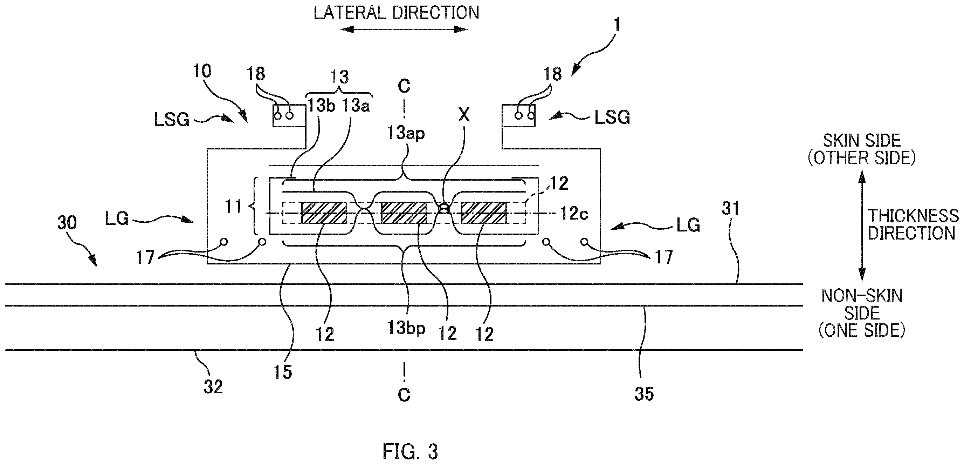

FIG. 1 is a schematic perspective view of the underpants-shaped disposable diaper 1. FIG. 2 is a schematic plan view of the diaper 1 in an unfolded and stretched state when viewed from a skin-side surface. FIG. 3 is a schematic cross-sectional view taken along arrows A-A in FIG. 2. The "unfolded state" is a state where the joining (welded portion SS) of side portions 30a of the front member 30 and side portions 40a of the back member 40, which are two side portions of the diaper 1, are separated from each other, and laid open such that the diaper 1 is entirely unfolded into a flat shape. The "stretched state" is a state where the elastic members of the diaper 1 have been stretched to an extent that wrinkles can no longer be seen in the diaper 1. Specifically, the stretched state is a state where the diaper 1 has been stretched such that the dimensions of the constituent members thereof (e.g., a later-described front member 30 and the like) match or are close to the dimensions of the members on their own. The line C-C in FIG. 2 and the like is a center line in the lateral direction, and the line CL is a center line in the longitudinal direction in an unfolded state. In FIG. 3, an adhesive is omitted for the sake of convenience. Further, for the sake of convenience, a dashed line indicates a visually-recognizable portion of an absorbent core 12 that is located on the front side in the longitudinal direction from the cross section. It should be noted that, in the following description, the diaper 1 in an unfolded and stretched state will be described, but the diaper 1 may be in a natural state.

As shown in FIG. 1, an underpants-shaped diaper 1 has a vertical direction, a lateral direction, and a front-back direction, and the diaper 1 includes a waist opening BH and a pair of leg openings LH formed therein. The vertical direction of the diaper 1 in the unfolded and stretched state in FIG. 2 will be referred to as a "longitudinal direction", the one side in the longitudinal direction will be referred to as a "front side" and the other side will be referred to as a "back side". In the front-back direction, the side corresponding to the wearer's stomach is the front side, and the side corresponding to the wearer's back is the back side. Further, the diaper 1 has a thickness direction as shown in FIG. 3, and with respect to the thickness direction, the side that comes into contact with the wearer is defined as the skin side, and the side opposite to the skin side is defined as the non-skin side.

The diaper 1 is a so-called three-piece type of diaper, and includes an absorbent main body 10, a front member 30, and a back member 40. The front member 30 and the back member 40 are substantially rectangular in a plan view, and the lengthwise direction thereof conforms to the lateral direction. The front member 30 covers the wearer's front side, and the back member 40 covers the wearer's back side. The absorbent main body 10 is substantially rectangular in a plan view. A front end portion and a back end portion of the absorbent main body 10 respectively overlap the skin-side surfaces of the front member 30 and the back member 40.

As shown in FIG. 2, the diaper 1 in an unfolded and stretched state is bilaterally symmetric about the center line C-C. The non-skin-side surfaces of the front end portion and the back end portion of the absorbent main body 10 are joined to the skin-side surfaces of the front member 30 and the back member 40 with use of an adhesive or the like (not shown), the absorbent main body 10 is folded one time so that the front member 30 and the back member 40 face each other, and two lateral side portions 30a of the front member 30 are joined on side weld portions SS by welding to two lateral side portions 40a of the back member 40, thus obtaining the diaper 1 in the underpants-shaped state.

The front member 30 and the back member 40 respectively include skin-side sheets 31 and 41 and non-skin-side sheets 32 and 42 that are made of soft nonwoven fabric or the like, and a plurality of elastic strings 35 and 45 that stretch and contract in the lateral direction. The plurality of elastic strings 35 and 45 are arranged side-by-side spaced apart in the vertical direction, and are fixed between the pairs of sheets (31 and 32, 41 and 42) in a state of being stretched in the lateral direction. Accordingly, the front member 30 and the back member 40 can stretch and contract in the lateral direction, and thus fit around the wearer's waist.

The skin-side sheet 31, the elastic strings 35, and the non-skin-side sheet 32 are overlaid on the skin side of the front member 30 in the thickness direction in this order, and these members are joined to each other with use of, for example, an adhesive such as a hot-melt adhesive. Similarly, the skin-side sheet 41, the elastic strings 45, and the non-skin-side sheet 42 are overlaid on the skin side of the back member 40 in the thickness direction in this order, and these members are joined to each other with use of, for example, an adhesive such as a hot-melt adhesive.

The skin-side sheets 31 and 41 and the non-skin-side sheets 32 and 42 are each a sheet made of nonwoven fabric, and specifically are made of spunbond nonwoven fabric sheets. However, there is no limitation thereto, and an SMS (spunbond/meltblown/spunbond) nonwoven fabric and the like may be used. It should be noted that the skin-side sheets 31 and 41 and the non-skin-side sheets 32 and 42 are not all required to be nonwoven fabric sheets, and a configuration is possible in which either the skin-side sheets 31 and 41 or the non-skin-side sheets 32 and 42 are soft sheet members not made of nonwoven fabric.

The absorbent main body 10 includes a top sheet 14, an absorbent body 11, and a back sheet 15, which are adhered using an adhesive such as a hot-melt adhesive. The top sheet 14 need only be a liquid-permeable sheet, and examples thereof include a hydrophilic air-through nonwoven fabric sheet and a spunbond nonwoven fabric sheet. The back sheet 15 need only be a liquid-impermeable sheet, and examples thereof include a polyethylene film, a polypropylene film, and a hydrophobic SMS nonwoven fabric. The top sheet 14 and the back sheet 15 has a size large enough to cover the entirety of the absorbent body 11.

The absorbent main body 10 includes leg gathers LG that are provided in the lateral end portions and stretch and contract in the longitudinal direction, and barrier cuffs LSG that are provided on the skin side with respect to the absorbent body 11 and serve as leak-proof wall portions for preventing lateral leakage. The leg gather LG and the barrier cuffs LSG respectively include elastic members 17 and elastic members 18 that stretch in the longitudinal direction (vertical direction).

### Absorbent body 11

The absorbent body 11 has a substantially rectangular shape in a plan view and has a laterally symmetric shape with respect to the center line C-C. The absorbent body 11 includes an absorbent core 12 that absorbs liquid, and a liquid-permeable core-wrapping sheet 13 that covers the absorbent core 12 from the skin side and the non-skin side in the thickness direction.

The liquid-absorbent material that constitutes the absorbent core 12 can be formed of liquid-absorbent fibers such as pulp fibers or liquid-absorbent granules such as superabsorbent polymer (so-called SAP), for example. In addition, the absorbent core may contain a liquid-absorbent material excluding the liquid-absorbent fibers or liquid-absorbent granules. In FIG. 3, a center 12c is the center in the thickness direction of the absorbent core 12.

The absorbent core 12 has a through hole 12H that penetrates in the thickness direction. As shown in FIG. 2 and the like, it is preferable that the absorbent core 12 has a laterally symmetric shape and has a pair of through holes 12H, and the shape of each through hole 12H is substantially the same. Further, it is preferable that the pair of through holes 12H are provided on the one side and the other side of the absorbent body 11 in the lateral direction, and at the central portion when the absorbent body 11 is divided into three portions in the longitudinal direction. This makes it easier to arrange the pair of through holes 12H at the position of the wearer's crotch while the diaper is put on, and therefore excrement is more likely to diffuse through the portion into which the absorbent core 12 is penetrated. Further, since the absorbent body 11 is more likely to fold from positions of the pair of through holes 12H, the absorbent body is likely to follow the movement of the wearer.

The longitudinal length of the through hole 12H is 30 to 500 mm, preferably 75 to 350 mm, and more preferably 100 to 250 mm. The lateral length of the through hole 12H is 3 to 20 mm, preferably 4 to 15 mm, and more preferably 5 to 12 mm. The through holes 12H of the present embodiment each have a substantially rectangular shape having a longitudinal length of 180 mm and a lateral length of 10 mm. As shown in FIG. 2, the through holes 12H each have a substantially rectangular shape extending along the longitudinal direction, and preferably have a shape extending along the longitudinal direction.

The diaper 1 includes a skin-side sheet 13a and a non-skin-side sheet 13b as the core-wrapping sheet 13. The lateral length of the skin-side sheet 13a is substantially equal to the lateral length of the absorbent core 12, and the longitudinal length thereof is longer than the longitudinal length of the absorbent core 12. The lateral length of the non-skin-side sheet 13b is longer than the lateral length of the absorbent core 12, and two lateral side end portions of the non-skin-side sheet 13b are folded back inward in the lateral direction, and are overlaid on the skin side with respect to the absorbent core 12 and on the skin side with respect to the skin-side sheet 13a.

Each of the skin-side sheet 13a and the non-skin-side sheet 13b is a sheet member formed of hydrophilic SMS nonwoven fabric. It should be noted that, as each of the skin-side sheet 13a and the non-skin-side sheet 13b, it is possible to use a hydrophilic water-permeable sheet member such as tissue, hydrophilic spunbond nonwoven fabric, hydrophilic air-through nonwoven fabric, or the like. The skin-side sheet 13a of the present embodiment has the thickness of approximately 1.0 mm, and two surfaces of the skin-side sheet 13a are substantially flat. A portion of the skin-side sheet 13a that covers the absorbent core 12 from the skin side in the thickness direction will be referred to as a "skin-side sheet portion 13ap", and a portion of the non-skin-side sheet 13b that covers the absorbent core 12 from the non-skin side in the thickness direction will be referred to as a "non-skin-side sheet portion 13bp".

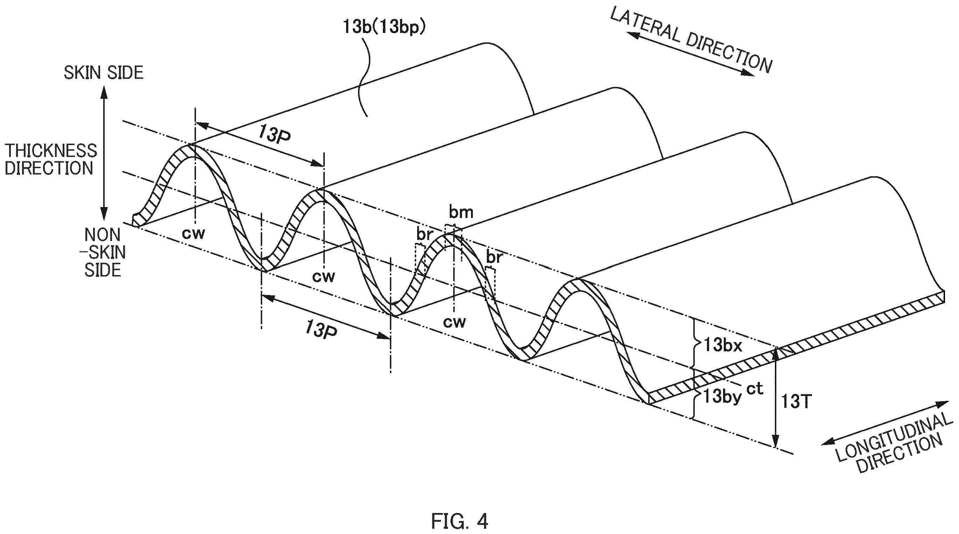

In the non-skin side sheet portion 13bp, a surface (skin-side surface) that faces the skin-side sheet portion 13ap has protruding portions (skin-side protruding portions 13bx) that protrude in the thickness direction (skin side). In the present embodiment, as shown in FIG. 4 and the like, the protruding portions that protrude in the thickness direction are provided on two surfaces of the non-skin-side sheet 13b. FIG. 4 is an explanatory diagram illustrating the non-skin-side sheet 13b. FIG. 4 is a cross-sectional view of a part of the non-skin-side sheet 13b shown in FIG. 3 or the like which is taken out and unfolded.

As shown in FIG. 4, the non-skin-side sheet 13b has a plurality of protruding portions that protrude toward the skin side or the non-skin side in the thickness direction. Specifically, the length of the non-skin-side sheet 13b in the thickness direction is a length 13T. Skin-side protruding portions 13bx and non-skin-side protruding portions 13by are provided across the entire region. The skin-side protruding portions 13bx are located on the skin side with respect to a center ct of the non-skin-side sheet 13b in the thickness direction, and protrude toward the skin side. The non-skin-side protruding portions 13by are located on the non-skin side with respect to the center ct and protrude toward the non-skin side. At this time, the skin-side protruding portions 13bx and the non-skin-side protruding portions 13by extend along the longitudinal direction. Therefore, when the non-skin-side sheet 13b is viewed from the skin side, the skin-side protruding portions 13bx that protrude toward the skin side and groove portions that are recessed toward the non-skin side (also referred to as "recessed portions") are provided alternately in the lateral direction. The groove portion recessed toward the non-skin side also serves as the non-skin-side protruding portion 13by that protrudes toward the non-skin side when the non-skin-side sheet 13b is viewed from the non-skin side. The skin-side protruding portions 13bx and the non-skin-side protruding portions 13by extend along the longitudinal direction. The lateral lengths of the skin-side protruding portions 13bx and the non-skin-side protruding portions 13by of the non-skin-side sheet 13b are equal. Further, among the skin-side protruding portions 13bx and the non-skin-side protruding portions 13by, which are provided alternately in the lateral direction, the skin-side protruding portions 13bx and the non-skin-side protruding portions 13by which are adjacent to each other in the lateral direction continue.

As shown in FIG. 4, in the non-skin-side sheet 13b, distances 13P between the lateral centers of the skin-side protruding portions 13bx located laterally-adjacent are equidistant, and distances 13P between the lateral centers of the plurality of non-skin-side protruding portions 13by are also equidistant.

It is preferable that, among the fibers that constitute the skin-side protruding portions 13bx, the fiber density of a lateral central portion bm of the skin-side protruding portion 13bx is higher than the fiber density of lateral side end portions br of the skin-side protruding portion 13bx. For example, in the non-skin-side sheet 13b of the present embodiment, the skin-side protruding portions 13bx and the non-skin-side protruding portions 13by are formed by passing the non-skin-side sheet 13b through between a pair of gear rolls that rotate while the outer circumferential surfaces of the rolls facing each other. The pair of gear rolls each have protruding portions and recessed portions on the outer circumferential surface, and are set so that the recessed portions of one gear roll and the protruding portions of the other gear roll engaged with each other. As shown in FIG. 4, the non-skin-side sheet 13b that has passed through the roll mechanism has a state where the skin-side protruding portions 13bx and the non-skin-side protruding portions 13by are arranged alternately along the lateral direction of the diaper 1. At this time, the side end portion br become a region which is stretched more than the central portion bm, and therefore the fiber density of the central portion bm becomes higher than the fiber density of the side end portion br. Similarly, the fiber density of the lateral central portion of the non-skin-side protruding portion 13by becomes higher than the fiber density of the lateral side end portion br of the non-skin-side protruding portion 13by.

As shown in FIG. 2, in a plan view, that is, when the diaper 1 (absorbent body 11) is viewed from the skin side in the thickness direction, the skin-side sheet portion 13ap and the non-skin-side sheet portion 13bp are joined inside each of the through holes 12H. By joining the skin-side sheet portion 13ap and the non-skin-side sheet portion 13bp inside each of the through holes 12H, it is possible to enhance the shape retention of the absorbent core 12 regardless of having the through holes 12H. The term "inside of the through hole 12H" refers to the inside of a region that is inside the through hole 12H in a plan view (when viewed from the skin side or non-skin side of the diaper 1) and is surrounded by edges that form the through hole 12H in a plan view.

As shown in FIG. 4 and the like, the central portion bm having a higher fiber density is located on the skin side with respect to the side end portion br. The central portion bm of the non-skin-side sheet 13b, which is located closer to the skin-side sheet 13a in the thickness direction, has a higher fiber density than the side end portion br. This makes it possible to further strengthen the joining between the skin-side sheet 13a and the non-skin-side sheet 13b inside the through hole 12H with an adhesive. Accordingly, since the joining between the skin-side sheet 13a and the non-skin-side sheet 13b inside the through holes 12H can be further strengthened, it is possible to enhance the shape retention of the absorbent core 12 against the movement of the wearer.

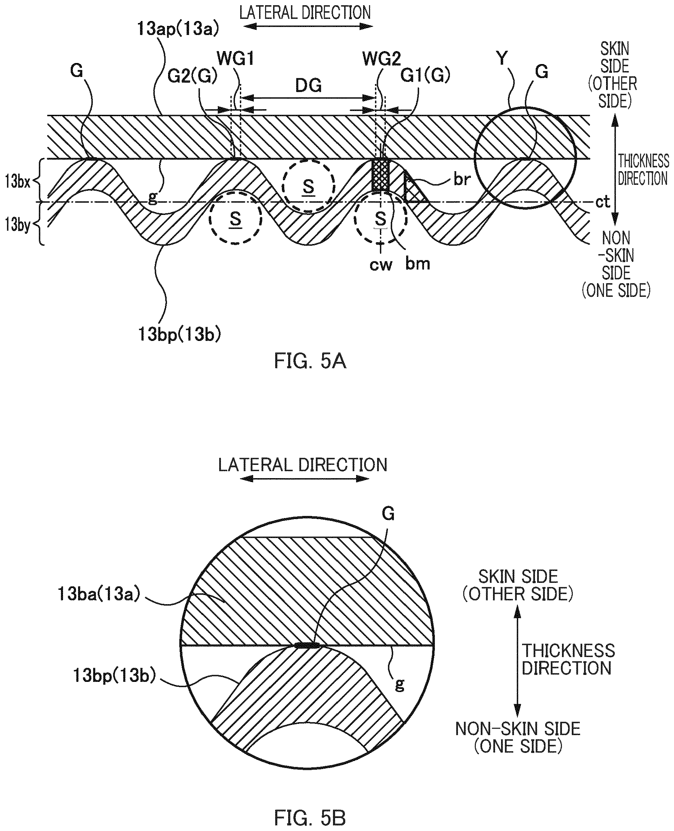

The following describes the joining inside the through hole 12H in a plan view. FIG. 5A is an enlarged view of a portion X in FIG. 3. FIG. 5B is an enlarged view of a portion Y in FIG. 5A. As shown in FIGS. 5A and 5B, inside the through hole 12H, the skin-side sheet portion 13ap and the non-skin-side sheet portion 13bp are joined with a plurality of joining portions G. Hereinafter, among the plurality of joining portions G provided inside the through hole 12H, a first joining portion G1 and a second joining portion G2 which are adjacent to each other in the lateral direction will be described.

Conventionally, it has been known that the through hole 12H is provided in the absorbent core 12 in order to enhance the followability of the absorbent core 12 to the wearer's body. Providing the through holes 12H causes a risk that the shape of the absorbent core 12 collapses. In this regard, in order to enhance the shape retention of the absorbent core 12, joining the skin-side sheet portion 13ap and the non-skin-side sheet portion 13bp of the core-wrapping sheet 13 that face each other, inside the through holes 12H is known. This joining between the skin-side sheet portion 13ap and the non-skin-side sheet portion 13bp is generally made using an adhesive such as a hot-melt adhesive, and this causes a risk of the decrease of the absorbency for excrement at the joining portion where the skin-side sheet portion 13ap and the non-skin-side sheet portion 13bp are joined.

In contrast, in the diaper 1 of the present embodiment, at least the first joining portion G1 and the second joining portion G2 are provided as the joining portions G inside the through hole 12H in a plan view. The first joining portion G1 and the second joining portion G2 are spaced apart from each other in the lateral direction by a predetermined distance (spacing distance DG). That is, inside the through hole 12H, there are portions in which the skin-side sheet portion 13ap and the non-skin-side sheet portion 13bp are joined (the first joining portion G1 and the second joining portion G2), and there are non-joining portions between the skin-side sheet portion 13ap and the non-skin-side sheet portion 13bp. Accordingly, while enhancing the followability of the absorbent core 12 along the wearer's body and the shape retention of the through hole 12H, the non-joining portion located between the first joining portion G1 and the second joining portion G2 in the lateral direction can reduce a risk that the absorption of excrement is hindered due to the joining between the skin-side sheet portion 13ap and the non-skin-side sheet portion 13bp. Further, a path for excrement is made easier to be provided between the first joining portion G1 and the second joining portion G2, facilitating the diffusion of excrement in the longitudinal direction. In particular, it is common that the longitudinal length of the absorbent core 12 of an absorbent article such as the diaper 1 is longer than the lateral length thereof. Therefore, in order to reduce the risk of the excrement leaking out from the lateral outer side, it is desirable that the excrement once absorbed by the absorbent core 12 is diffused in the longitudinal direction rather than in the lateral direction. Therefore, since the first joining portion G1 and the second joining portion G2 are spaced apart from each other in the lateral direction, the absorbed excrement is more likely to diffuse in the longitudinal direction, making it possible to reduce the risk that the excrement leaks outside of the absorbent core 12 (diaper 1) in the lateral direction.

In a configuration in which the first joining portion G1 and the second joining portion G2 are spaced apart from each other in the lateral direction by a predetermined distance, the diaper 1 has the skin-side protruding portions (first sheet protruding portions) 13bx that protrude toward the skin side in the thickness direction, on a surface of the non-skin-side sheet portion (first sheet portion) 13bp that faces the skin-side sheet portion (second sheet portion) 13ap. In addition, the skin-side protruding portions 13bx of the non-skin-side sheet portion 13bp and the skin-side sheet portion 13ap are joined. Specifically, as shown in FIG. 4, the non-skin-side sheet 13b includes the skin-side protruding portions 13bx that protrude toward the skin side. Further, in this non-skin-side sheet 13b, inside the through hole 12H, as shown in FIG. 5A, the non-skin-side sheet portion 13bp has the plurality of skin-side protruding portions 13bx that protrude in the thickness direction, on the surface that faces the skin-side sheet portion 13ap, and the skin-side protruding portions 13bx and the skin-side sheet portion 13ap are joined to each other. The first joining portion G1 and the second joining portion G2 which are spaced apart from each other in the lateral direction are provided due to the skin-side protruding portions 13bx of the non-skin-side sheet portion 13bp, and therefore spaces S between the skin-side sheet portion 13ap and the non-skin-side sheet portion 13bp are provided, making the excrement absorbed from the skin side likely to remain in the space S. Accordingly, this makes it possible to reduce the risk that the excrement once absorbed by the absorbent body 11 returns to the skin side and attaches to the wearer's skin.

At this time, closest-to-the-skin-side portions (apexes) of the skin-side protruding portions 13bx of the non-skin-side sheet portion 13bp are each joined to the skin-side sheet portion 13ap, forming the first joining portion G1 and the second joining portion G2. On the other hand, in the non-skin-side sheet portion 13bp, between the skin-side protruding portions 13bx that are adjacent to each other in the lateral direction, the non-skin-side protruding portions 13by that protrude toward the non-skin side are provided, and the non-skin-side protruding portions 13by become groove portions that are recessed toward the non-skin side when viewed from the skin side. Therefore, as shown in FIG. 5A, between the first joining portion G1 and the second joining portion G2 which are spaced apart from each other by a predetermined distance in the lateral direction, the space S is formed between the skin-side sheet portion 13ap and the non-skin-side sheet portion 13bp. This space S makes excrement received in the through hole 12H more likely to remain in the space S provided between the first joining portion G1 and the second joining portion G2 in the lateral direction, making it possible to alleviate a decrease in absorbency due to the joining between the skin-side sheet portion 13ap and the non-skin-side sheet portion 13bp. Further, this space S makes it more likely to diffuse the absorbed excrement in the longitudinal direction.

It is preferable that, inside the through hole 12H, the average height of the protruding portions on the surface of the skin-side sheet portion 13ap that faces the non-skin-side sheet portion 13bp is lower than the average height of the skin-side protruding portions 13bx of the non-skin-side sheet portion 13bp. The height of the protruding portion refers to the length from the center of each sheet portion in the thickness direction to the apex of the protruding portion. For example, the height of the skin-side protruding portion 13bx of the non-skin-side sheet portion 13bp is the length from the center ct of the height 13T of the non-skin-side sheet portion 13bp in the thickness direction to the skin-side apex of the skin-side protruding portion 13bx in the thickness direction. The height of the non-skin-side protruding portion 13by is the length from the center ct of the height 13T of the non-skin-side sheet portion 13bp in the thickness direction to the non-skin-side apex of the non-skin-side protruding portion 13by in the thickness direction. On the other hand, since the surface of the skin-side sheet portion 13ap is substantially flat, the skin-side sheet portion does not have a portion that protrudes toward the skin side or the non-skin side, and the average height of the protruding portions is zero. That is, in the diaper 1, on the surface of the skin-side sheet portion 13ap that faces the non-skin-side sheet portion 13bp, the average height of the protruding portions is zero, and therefore it is clear that the average height of the protruding portions is lower than the average height of the skin-side protruding portions 13bx of the non-skin-side sheet portion 13bp,. Accordingly, compared with the case where the skin-side sheet portion 13ap includes protruding portions in the same manner as the skin-side protruding portions 13bx of the non-skin-side sheet portion 13bp, the skin-side sheet portion 13ap and the skin-side protruding portions 13bx can be more reliably brought into contact each other, and it is possible to further strengthen the joining between the skin-side sheet portion 13ap and the non-skin-side sheet portion 13bp in the first joining portion G1 and the second joining portion G2.

Further, as shown in FIGS. 4, 5, and the like, it is preferable that the non-skin-side sheet portion 13bp has the plurality of protruding portions 13bx and 13by not only on the surface that faces the skin-side sheet portion 13ap but also on two side surfaces. As described above, the non-skin-side sheet 13b of the diaper 1 pass through between the pair of rolls in which the protruding portions of one gear roll and the recessed portions of the other gear roll are engaged with each other, and therefore the protruding portions 13bx and 13by are formed on two side surfaces. It should be noted that the height of the plurality of skin-side protruding portions 13bx of the diaper 1 formed in this manner is substantially the same as the height of the plurality of non-skin-side protruding portions 13by. By providing not only the skin-side protruding portions 13bx that face the skin-side sheet portion 13ap but also the non-skin-side protruding portions 13by that protrude toward the non-skin side, the space S can be provided not only between the skin-side sheet portion 13ap and the non-skin-side sheet portion 13bp but also in a portion on the non-skin side with respect to the non-skin-side sheet portion 13bp. This makes excrement more likely to remain in the space S and to diffuse in the longitudinal direction in the space S.

Further, as shown in FIG. 3, in the case where the skin-side sheet portion 13ap is substantially flat and the non-skin-side sheet portion 13bp has the protruding portions 13bx and 13by that protrude on two sides in the thickness direction, it is preferable that the joining portions G (first joining portion G1 and second joining portion G2) for joining the skin-side sheet portion 13ap and the non-skin-side sheet portion 13bp are provided on the skin side with respect to a center 12c of the absorbent core 12 in the thickness direction. Accordingly, the through hole 12H of the absorbent body 11 is recessed deeper toward the skin side from the non-skin-side surface, and therefore the wearer or the like is more likely to recognize the presence of the through hole 12H when viewed from the non-skin-side surface of the diaper 1. In the diaper 1, the sheet pass through between the pair of rolls in which the protruding portions of one gear roll and the recessed portions of the other gear roll are engaged with each other, and therefore the protruding portions 13bx and 13by are formed on two surfaces of the non-skin-side sheet 13b. The non-skin-side sheet 13b formed in this manner has a higher lateral stretchability than the skin-side sheet 13a. Therefore, when the skin-side sheet 13a and the non-skin-side sheet 13b are respectively arranged on the skin side and the non-skin side of the absorbent core 12, the non-skin-side sheet 13b is arranged up to the skin side with respect to the center 12c of the absorbent core 12 in the thickness direction, and the joining portion G is positioned on the skin side with respect to the center 12c of the absorbent core 12 in the thickness direction.

It is more preferable that inside the through hole 12H, in addition to the first joining portion G1 and the second joining portion G2, a joining portion G (another joining portion) in which the skin-side sheet portion 13ap and the non-skin-side sheet portion 13bp are joined is provided, and the other joining portion G is spaced apart from both of the first joining portion G1 and the second joining portion G2. That is, it is preferable that three or more joining portions G are provided inside the through hole 12H, being spaced apart from each other. As shown in FIG. 5, in the diaper 1, there are provided four or more joining portions G including the first joining portion G1 and the second joining portion G2 for joining the skin-side sheet portion 13ap and the non-skin-side sheet portion 13bp. Accordingly, the joining between the skin-side sheet portion 13ap and the non-skin-side sheet portion 13bp inside the through hole 12H can be further strengthened, and a plurality of spaces S can be provided which are made by separating the laterally adjacent joining portions apart from each other by a predetermined distance. This makes excrement more likely to diffuse in the longitudinal direction while enhancing the absorbency in the through hole 12H.

As shown in FIG. 4, in the non-skin-side sheet portion 13bp of the diaper 1, the plurality of skin-side protruding portions 13bx extending along the longitudinal direction are provided in the lateral direction. In the non-skin-side sheet portion 13bp, two skin-side protruding portions 13bx that are adjacent to each other in the lateral direction each are in contact with the skin-side sheet portion 13ap. In the joining between the non-skin-side sheet portion 13bp and the skin-side sheet portion 13ap inside the through hole 12H, it is preferable that, as shown in FIG. 2, the shape of the through hole 12H extends along the longitudinal direction, and it is preferable that two laterally adjacent protruding portions are separated apart from each other in the lateral direction and extend along the longitudinal direction. The skin-side apex of the skin-side protruding portion 13bx is in contact with the skin-side sheet portion 13ap. The portion where an adhesive g provided on the skin-side sheet portion 13ap is in contact with and is joined to the skin-side protruding portion bx is the joining portion G. Since the shape of the through hole 12H extends along the longitudinal direction, and two protruding portions that are spaced apart in the lateral direction extend along the longitudinal direction, this makes the space S between the first joining portion G1 and the second joining portion G2 in the lateral direction likely to become a region that extends along the longitudinal direction, making excrement more likely to diffuse in the longitudinal direction inside the through hole 12H. As a result, in the absorbent body 11, it is possible to reduce the risk that excrement is diffused in the lateral direction of the absorbent body 11 and excrement leaks outside of the absorbent body 11 (diaper 1) in the lateral direction.

Further, it is more preferable that, inside the through hole 12H, the longitudinal distance at which two skin-side protruding portions 13bx extending along the longitudinal direction are in contact with the skin-side sheet portion 13ap is longer than the spacing distance (predetermined distance) DG between the first joining portion and the second joining portion in the lateral direction. Accordingly, compared with the case where the longitudinal distance at which the two skin-side protruding portions 13bx are in contact with the skin-side sheet portion 13ap is equal to or smaller than the spacing distance DG, the space S provided between the first joining portion G1 and the second joining portion G2 in the lateral direction is made more likely to maintain its shape extending along the longitudinal direction, inside the through hole 12H. This make the absorbed excrement more likely to diffuse along the longitudinal direction.

Regarding the first joining portion G1 and the second joining portion G2, it is preferable that, as shown in FIG. 5A, the spacing distance DG between the first joining portion G1 and the second joining portion G2 in the lateral direction is longer than the lateral length obtained by dividing by two the sum of the lateral length WG1 of the first joining portion G1 and the lateral length WG2 of the second joining portion G2 (DG > (WG1 + WG2)/2). In such a case, compared with the case where the spacing distance DG is shorter than the value obtained by dividing by two the sum of the lengths WG1 and WG2, it is possible to narrow the regions of the first joining portion G1 and the second joining portion G2. This can decrease the stiffness inside the through hole 12H, and reduces the risk that the joining portions G prevents the absorbent body 11 from being deformed from the through holes 12H, making the absorbent body 11 likely to be deformed along the wearer's body.

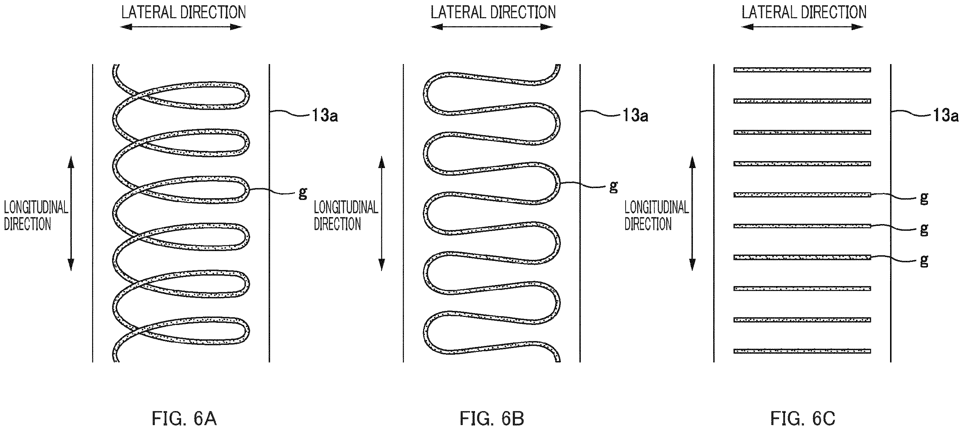

In the diaper 1, the joining portions G (the first joining portion G1 and the second joining portion G2) are joined with an adhesive such as a hot-melt adhesive. The adhesive that forms the joining portion G is provided on the skin-side sheet 13a or the non-skin-side sheet 13b, and in the present embodiment, as shown in FIG. 6A, the hot-melt adhesive g is provide on the skin-side sheet 13a. FIG. 6A is an explanatory diagram illustrating an application region of an adhesive g of the diaper 1. FIG. 6A shows a state of a part of the skin-side sheet 13a when viewed from the non-skin side. It is preferable that the adhesive g is provided in a manner of continuing in the lateral direction. The application pattern of the adhesive g shown in FIG. 6A is a so-called spiral pattern, and the adhesive g is provided in a manner of continuing in the lateral direction. Accordingly, by making the adhesive g more likely to be arranged in the portions where the skin-side sheet portion 13ap and the non-skin-side sheet portion 13bp are in contact with each other, this makes it possible to form the first joining portion G1 and the second joining portion G2. As a result, it is possible to more reliably form a region (space S) located between the first joining portion G1 and the second joining portion G2 where they are separated apart. The absorbency for excrement is enhanced, and excrement is more likely to diffuse in the longitudinal direction.

It is sufficient that the adhesive for forming the joining portion G is provided on at least either one of the skin-side sheet 13a and the non-skin-side sheet 13b. The adhesive may be provided on any of these sheets. However, as in the present embodiment, in the case where one is a flat sheet (skin-side sheet 13a) and the other is a sheet having protrusions and recesses (non-skin-side sheet 13b), it is preferable that the adhesive is applied to the flat sheet. Accordingly, the flat sheet and the protruding portions of the sheet having protrusions and recesses can be more reliably joined. In the present embodiment, the joining portion G is provided by using an adhesive such as a hot-melt adhesive. But the joining portion G is not necessarily limited to the portion formed by the adhesive. For example, welding and compression may be adopted.

It should be noted that the application pattern of the adhesive g provided on the skin-side sheet 13a or the non-skin-side sheet 13b is not limited to the so-called spiral pattern shown in FIG. 6A. FIGS. 6B and 6C are explanatory diagrams illustrating application regions of the adhesive g of the diaper 1 in another embodiment. FIG. 6B is a so-called Ω-shaped pattern, and is an application pattern of the adhesive g provided in a state where a plurality of rows of the adhesive g extending along the lateral direction are continuous in the longitudinal direction. FIG. 6C is an application pattern of the adhesive g in which a plurality of adhesives g extending along the lateral direction are arranged intermittently in the longitudinal direction. As shown in FIGS. 6A to 6C, by providing the adhesive g in a state of being arranged in a manner of continuing in the lateral direction, the skin-side protruding portions 13bx of the non-skin-side sheet portion 13bp can be joined to the skin-side sheet portion 13ap, making it possible to further strengthen the joining between the first joining portion G1 and the second joining portion G2 inside the through hole 12H. Further, as the application pattern of the adhesive g to the skin-side sheet 13a or the non-skin-side sheet 13b, there may be used a pattern in which the adhesive g is applied to the entire region of the skin-side sheet 13a or the non-skin-side sheet 13b without any gaps (so-called solid application).

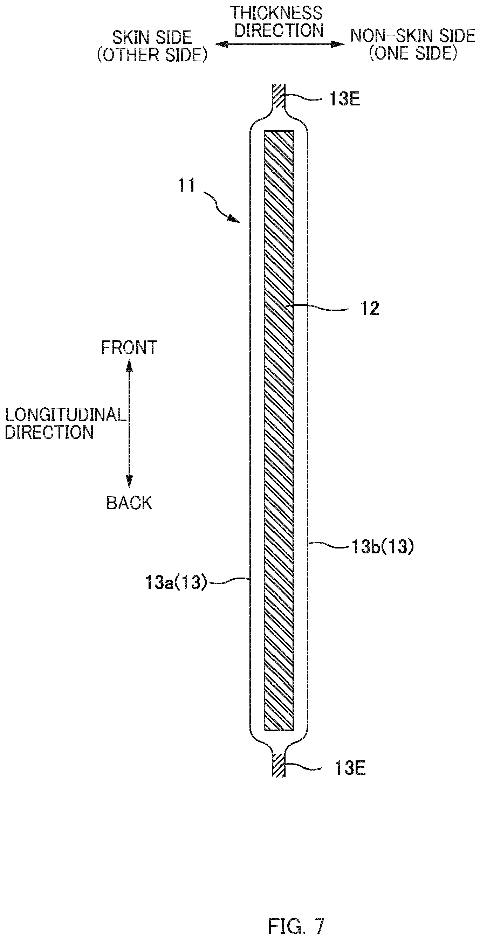

It is preferable that in the absorbent body 11, at least the longitudinal one-side ends of the skin-side sheet 13a and the non-skin-side sheet 13b are positioned outside the longitudinal one-side end of the absorbent core 12, and on the one side in the longitudinal direction, the end portion of the skin-side sheet 13a and the end portion of the non-skin-side sheet 13b are joined continuing in the lateral direction. In the diaper 1, as shown in FIGS. 1 and 7, the front end of the skin-side sheet 13a and the front end of the non-skin-side sheet 13b in the longitudinal direction are each positioned outside the front end of the absorbent core 12, and the back end of the skin-side sheet 13a and the back end of the non-skin-side sheet 13b are each positioned outside the back end of the absorbent core 12. That is, the longitudinal length of each of the skin-side sheet 13a and the non-skin-side sheet 13b is longer than the longitudinal length of the absorbent core 12, and the longitudinal length of each of the skin-side sheet portion 13ap and the non-skin-side sheet portion 13bp is longer than the longitudinal length of the absorbent core 12. At this time, on the front side in the longitudinal direction, the front end portion of the skin-side sheet portion 13ap and the front end portion of the non-skin-side sheet portion 13bp are joined in a joining portion 13E. Similarly, on the back side in the longitudinal direction, the back end portion of the skin-side sheet portion 13ap and the back end portion of the non-skin-side sheet portion 13bp are joined in another joining portion 13E. In each of the joining portions 13E, the skin-side sheet portion 13ap and the non-skin-side sheet portion 13bp are joined to each other by the application of heat and pressure. The joining portions 13E are joined in a state of being continuous in the lateral direction, and are joined in a state of being continuous in the front end portion and the back end portion from the lateral one-side end portion to the lateral other-side end portion of the core-wrapping sheet 13. By providing the joining portions 13E, excrement is dammed by the joining portions 13E provided outside the end portion of the absorbent core 12 in the longitudinal direction, even when the excrement is diffused in the longitudinal direction through the space S located between the first joining portion G1 and the second joining portion G2 and reaches any of the longitudinal end portions of the absorbent core 12. This makes it possible to reduce the risk that the excrement leaks out from the longitudinal outer side of the absorbent body 11. It should be noted that, in the diaper 1, the joining portions 13E are provided in the longitudinal front and back end portions of the absorbent body 11. However, the joining portions 13E may be provided in either of the front end portion and the back end portion.

### Other Embodiments

Although the above embodiments of the present disclosure are simply to facilitate understanding of the present disclosure and are not in any way to be construed as limiting the present disclosure. The present disclosure may variously be changed or altered without departing from its gist and encompass equivalents thereof.

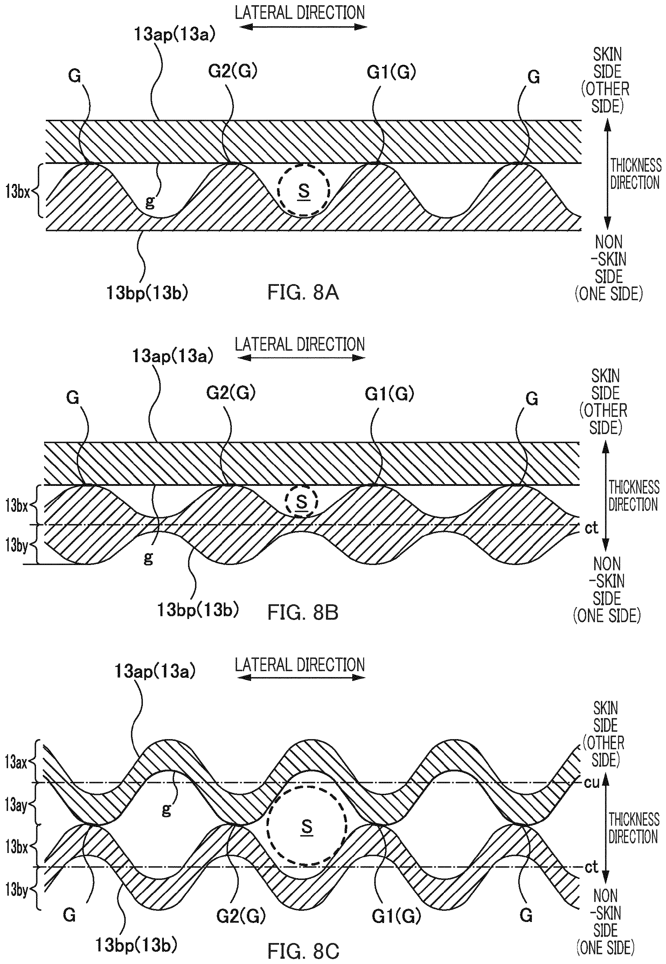

In the above-described embodiment, in the through hole 12H, the skin-side sheet portion 13ap having a skin-side surface and a non-skin-side surface which are flat, and the non-skin-side sheet portion 13bp having a skin-side surface and a non-skin-side surface which respectively include the protruding portions 13bx and 13by are joined, but the configuration is not limited thereto. FIGS. 8A to 8C are enlarged views of portions respectively according to another embodiments, corresponding to portion X in FIG. 3. As shown in FIG. 8A, the configuration is as follow: the skin-side sheet portion 13ap (second sheet portion) having flat two surfaces and the skin-side protruding portions 13bx provided on the surface (skin-side surface) of the non-skin-side sheet portion 13bp (first sheet portion) that faces the skin-side sheet portion 13ap (second sheet portion) are joined. The non-skin-side surface of the non-skin-side sheet portion 13bp may be substantially flat. Further, as shown in FIG. 8B, a configuration is also acceptable in which the skin-side sheet portion 13ap (second sheet portion) having flat two surfaces and the skin-side protruding portions 13bx provided on the surface (skin-side surface) of the non-skin-side sheet portion 13bp (first sheet portion) that faces the skin-side sheet portion 13ap (second sheet portion) are joined, and in which the non-skin-side protruding portions 13by are provided at the positions that overlap the skin-side protruding portions 13bx of the non-skin-side sheet portion 13bp with respect to the lateral direction. Also in the configurations shown in FIGS. 8A and 8B, the space S is likely to be provided between the first joining portion G1 and the second joining portion G2 in the lateral direction, and therefore excrement is likely to be absorbed even in the through hole 12H, making the absorbed excrement likely to diffuse in the longitudinal direction.

In the above-described embodiment, the skin-side sheet portion 13ap is a flat sheet, and the non-skin-side sheet portion 13bp includes the skin-side protruding portions 13bx. But the configuration is not limited thereto. For example, as shown in FIG. 8C, the following configuration is acceptable: in addition that the non-skin-side sheet portion 13bp has the skin-side protruding portions 13bx on the surface that faces the skin-side sheet portion 13ap, the skin-side sheet portion 13ap has the non-skin-side protruding portions (second sheet protruding portions) 13ay on the surface that faces the non-skin-side sheet portion 13bp. In such a case, by joining the non-skin-side protruding portions 13ay of the skin-side sheet portion 13ap and the skin-side protruding portions 13bx of the non-skin-side sheet portion 13bp in the joining portions G inside the through hole 12H, it is possible to provide the space S between the first joining portion G1 and the second joining portion G2 in the lateral direction. This enhances the absorbency for excrement and makes excrement more likely to diffuse in the longitudinal direction. Further, the skin-side sheet portion 13ap has protruding portions as well as the non-skin-side sheet portion 13bp has, and therefore the thickness of the core-wrapping sheet 13 inside the through hole 12H can be increased. This makes excrement more likely to remain, making it possible to reduce the risk that the absorbed excrement returns to the skin side. It should be noted that, as a configuration in which the skin-side sheet portion 13ap has protruding portions as well as the non-skin-side sheet portion 13bp has, it is not necessarily required that, as shown in FIG. 8C, the skin-side sheet portion 13ap includes the skin-side protruding portions 13ax and the non-skin-side protruding portions 13ay, and the non-skin-side sheet portion 13bp include both the skin-side protruding portions 13bx and the non-skin-side protruding portions 13by. For example, a configuration may be adopted in which the skin-side surface of the skin-side sheet portion 13ap and the non-skin-side surface of the non-skin-side sheet portion 13bp are flat.

In the above-described embodiment, the core-wrapping sheet 13 is formed of two sheets including the skin-side sheet 13a and the non-skin-side sheet 13b, but the configuration is not limited thereto. For example, the core-wrapping sheet 13 may have a configuration in which one sheet serves as the skin-side sheet portion 13ap and the non-skin-side sheet portion 13bp, and the core-wrapping sheet 13 may have a configuration in which three or more sheets serve as the skin-side sheet portion 13ap and the non-skin-side sheet portion 13bp.

In the above-described embodiment, regarding the first joining portion G1 and the second joining portion G2, the spacing distance DG between the first joining portion G1 and the second joining portion G2 in the lateral direction is longer than the lateral length obtained by dividing by two the sum of the lateral length WG1 of the first joining portion G1 and the lateral length WG2 of the second joining portion G2 (DG > (WG1 + WG2)/2). But the configuration is not limited thereto. The spacing distance DG between the first joining portion G1 and the second joining portion G2 in the lateral direction may be shorter than the lateral length obtained by dividing by two the sum of the lateral length WG1 of the first joining portion G1 and the lateral length WG2 of the second joining portion G2 (DG < (WG1 + WG2)/2). Accordingly, compared with the case where the spacing distance DG is longer than the value obtained by dividing by two the sum of the lengths WG1 and WG2, it is possible to make larger the regions of the joining portions G. This can further strengthen the joining between the skin-side sheet portion 13ap and the non-skin-side sheet portion 13bp inside the through hole 12H.

Further, in the above-described embodiment, the joining portions G (the first joining portion G1 and the second joining portion G2) are provided on the skin side with respect to the center 12c of the absorbent core 12 in the thickness direction. But the configuration is not limited thereto. The joining portion G may be provided on the non-skin side with respect to the central portion in the thickness direction or in the center 12c of the absorbent core 12 in the thickness direction. For example, in the process of forming the joining portion G, the following method is acceptable: in a state where the skin-side sheet portion 13ap and the non-skin-side sheet portion 13bp are arranged on two surfaces of the absorbent core 12, the absorbent core 12 is compressed (pressed with a pin or the like) from the skin side of the through hole 12H or the non-skin side, and in this state the skin-side sheet portion 13ap and the non-skin-side sheet portion 13bp are joined to each other inside the through hole 12H in a plan view. In this case, in the case where compression is applied from the skin side of the through hole 12H, the joining portion G is provided on the non-skin side with respect to the center 12c of the absorbent core 12 in the thickness direction. In the case where compression is applied from both the skin side and the non-skin side of the through hole 12H, the joining portion G is provided at the central portion of the absorbent core 12 in the thickness direction.

In the above-described embodiment, the non-skin-side sheet 13b has the skin-side protruding portions 13bx and the non-skin-side protruding portions 13by across the entire region, but the configuration is not limited thereto. The skin-side protruding portions 13bx may be provided only in a portion of the non-skin-side sheet portion 13bp that is located at least inside the through hole 12H.

### REFERENCE SIGNS LIST

1: diaper (underpants-shaped disposable diaper, absorbent article),
10: absorbent main body,
11: absorbent body,
12: absorbent core,
12H: through hole,
13: core-wrapping sheet,
13a: skin-side sheet,
13ap: skin-side sheet portion (first sheet portion),
13b: non-skin-side sheet
13bp: non-skin-side sheet portion (second sheet portion),
13bx: skin-side protruding portion (first sheet protruding portion),
13by: non-skin-side protruding portion,
13E: joining portion,
14: top sheet,
15: back sheet,
17: elastic member,
18: elastic member,
30: front member,
31: skin-side sheet,
32: non-skin-side sheet,
35: elastic string,
40: back member,
41: skin-side sheet,
42: non-skin-side sheet,
45: elastic string,
g: adhesive,
G: joining portion,
S: space,
LH: leg opening,
BH: waist opening

## Claims

1. An absorbent article having a longitudinal direction, a lateral direction, and a thickness direction that are orthogonal to each other,
the absorbent article comprising:
an absorbent body including
an absorbent core,
a first sheet portion that covers the absorbent core from a one side in the thickness direction, and
a second sheet portion that covers the absorbent core from another side in the thickness direction,
the absorbent core including a through hole that penetrates in the thickness direction,
the absorbent article has a first joining portion and a second joining portion inside the through hole in a plan view,
each of the first joining portion and the second joining portion being a portion in which the first sheet portion and the second sheet portion are joined,
the second joining portion being spaced apart from the first joining portion in the lateral direction by a predetermined distance in the plan view.

2. The absorbent article according to claim 1, wherein
inside the through hole, the first sheet portion has a plurality of protruding portions protruding in the thickness direction, at least on a surface of the first sheet portion that faces the second sheet portion, and
the protruding portions and the second sheet portion are joined by each of the first joining portion and the second joining portion.

3. The absorbent article according to claim 2, wherein
the first sheet portion has the plurality of protruding portions on two side surfaces in the thickness direction.

4. The absorbent article according to claim 3, wherein
a fiber density of a central portion of the protruding portion in the lateral direction is higher than a fiber density of a side end portion of the protruding portion in the lateral direction.

5. The absorbent article according to claim 3 or 4, wherein
inside the through hole,
an average height of the protruding portions located on a surface of the second sheet portion that faces the first sheet portion
is lower than
an average height of the protruding portions located on a surface of the first sheet portion that faces the second sheet portion.

6. The absorbent article according to claim 1, wherein
inside the through hole in the plan view, the first sheet portion has a plurality of first sheet protruding portions protruding in the thickness direction, on a surface of the first sheet portion that faces the second sheet portion,
inside the through hole in the plan view, the second sheet portion has a plurality of second sheet protruding portions protruding in the thickness direction, at least on a surface of the second sheet portion that faces the first sheet portion, and
the first sheet protruding portions and the second sheet portion are joined by each of the first joining portion and the second joining portion, or the second sheet protruding portions and the first sheet portion are joined by each of the first joining portion and the second joining portion.

7. The absorbent article according to any one of claims 1 to 6, wherein
inside the through hole, the absorbent article has
the first joining portion,
the second joining portion, and
another joining portion in which, and at least one or more portions of the first sheet portion and the second sheet portion are joined, and
the other joining portion is spaced apart from the first joining portion and the second joining portion in the lateral direction.

8. The absorbent article according to any one of claims 1 to 7, wherein
the through hole extends along the longitudinal direction,
inside the through hole, the first sheet portion has a plurality of protruding portions on a surface of the first sheet portion that faces at least the second sheet portion,
the plurality of protruding portions coming into contact with the second sheet portion and protruding in the thickness direction, and
two protruding portions that are laterally adjacent to each other are spaced apart in the lateral direction and extend along the longitudinal direction.

9. The absorbent article according to claim 8, wherein
inside the through hole, a longitudinal distance at which the two protruding portions are in contact with the second sheet portion is longer than the predetermined distance.

10. The absorbent article according to any one of claims 1 to 9, wherein
the one side in the thickness direction is a non-skin side,
the other side in the thickness direction is a skin side, and
the first sheet portion is a non-skin-side sheet portion that is located on the non-skin side with respect to the absorbent core.

11. The absorbent article according to claim 10, wherein
the first joining portion and the second joining portion are provided on the skin side with respect to a center of the absorbent core in the thickness direction.

12. The absorbent article according to any one of claims 1 to 11, wherein
an adhesive is provided in at least either one of the first sheet portion and the second sheet portion, and
the adhesive is provided in a manner of continuing in the lateral direction.

13. The absorbent article according to any one of claims 1 to 12, wherein in the lateral direction,
a spacing distance between the first joining portion and the second joining portion is shorter than a length obtained by dividing by two a sum of a length of the first joining portion and a length of the second joining portion.

14. The absorbent article according to any one of claims 1 to 12, wherein in the lateral direction,
a spacing distance between the first joining portion and the second joining portion is longer than a length obtained by dividing by two a sum of a length of the first joining portion and a length of the second joining portion.

15. The absorbent article according to any one of claims 1 to 14, wherein
at least longitudinal one-side ends of the first sheet portion and the second sheet portion are positioned outside a longitudinal one-side end of the absorbent core, and
on a one side in the longitudinal direction,
an end portion of the first sheet portion and an end portion of the second sheet portion are joined continuing in the lateral direction.

16. The absorbent article according to any one of claims 1 to 15, wherein
the absorbent body includes a pair of the through holes at a central portion when the absorbent body is divided into three portions in the longitudinal direction, and
the pair of through holes are provided on a one side and another side of the absorbent body in the lateral direction.
